Europäisches Patentamt

European Patent Office

Office européen des brevets

(19)

(11) Veröffentlichungsnummer: **0 329 696**
**B1**

(12) **EUROPÄISCHE PATENTSCHRIFT**

(45) Veröffentlichungstag der Patentschrift: **09.01.91**

(51) Int. Cl.⁵: **A 61 K 6/06,** A 61 K 6/08

(21) Anmeldenummer: **87907508.3**

(22) Anmeldetag: **02.11.87**

(86) Internationale Anmeldenummer:
**PCT/EP87/00657**

(87) Internationale Veröffentlichungsnummer:
**WO 88/03405 19.05.88 Gazette 88/11**

(54) **TEILPROTHETISCHES VERBINDUNGSELEMENT AUS METALL.**

(30) Priorität: **06.11.86 CH 4434/86**

(43) Veröffentlichungstag der Anmeldung:
**30.08.89 Patentblatt 89/35**

(45) Bekanntmachung des Hinweises auf die
Patenterteilung:
**09.01.91 Patentblatt 91/02**

(84) Benannte Vertragsstaaten:
**AT BE CH DE FR GB IT LI LU NL SE**

(56) Entgegenhaltungen:
**EP-A-0 151 233**
**DE-A-1 594 120**
**DE-A-2 723 339**
**FR-A-2 342 327**
**US-A-4 113 792**
**US-A-4 364 731**

(73) Patentinhaber: **SCHUTZ- DENTAL GMBH**
**Homburger Strasse 64**
**D-6365 Rosbach 1 (DE)**

(72) Erfinder: **SCHÜTZ, Horst**
**Homburger Strasse 64**
**D-6365 Rosbach 1 (DE)**

(74) Vertreter: **Lusuardi, Werther Giovanni**
**Dr. Lusuardi AG Kreuzbühlstrasse 8**
**CH-8008 Zürich (CH)**

Courier Press, Leamington Spa, England.

**Beschreibung**

Die Erfindung bezieht sich auf ein teilprothetisches Verbindungselement aus Metall, insbesondere Geschiebe, für die Dentaltechnik, mit einer mindestens teilweisen Beschichtung der Metalloberfläche mit einer Haftvermittlerschicht zur Verbindung mit dentalen Kunststoffen.

Teilprothetische Verbindungselemente dienen der Verbindung zwischen Restzähnen und Ersatzzähnen und weisen eine positiven (Patrize) und einen diesen umfassenden negativen Teil (Matrize) auf, die je nach dem Ort, wo das Verbindungselement angebracht werden und nach der Funktion, die es erfüllen soll, geformt sind.

Das Problem der Verbindung zwischen dentalem kunststoff und Metallen in der Zahntechnik ist nicht neu. Die bisherigen Lösungsversuche betreffen jedoch lediglich Metall-Kunststoff-Verblendungen, welche kaum Kräfte zu übertragen haben und ausschliesslich ästhetischen Zwecken dienen. Die bisher bekannt gewordenen Verblendungen mit einer Kunststoff-Metall-Haftvermittlerschicht weisen denn auch, insbesondere nach Verbringung ins Mundmilieu, extrem niedrige Scherfestigkeitswerte auf, die zudem eine grosse Streuung aufweisen. Es kann daraus geschlossen werden, dass nur eine schwache chemische Bindung zwischen Metall, Haftvermittlerschicht und dentalem Kunststoff erreicht wird.

Aus der US—A—4.364.731 ist beispielsweise eine Komposit-Zahnkrone bekannt, bei der auf der Metalloberfläche eine erste, exogene Beschichtung aus einem anorganischen Oxid und eine, nachträglich aufgebrachte, zweite Beschichtung mit einem Silanhaftvermittler aufgebracht Wurde. Die spätere Verbindung der solcherart aufgebauten Haftvermittlerschicht mit dem dentalen Kunststoff, kann wegen der ungenügenden chemischen Verwandschaft keine guten Haftwerte ergeben, was durch die ermittelten Scherfestigkeitswerte bestätigt wird.

Hier will die Erfindung Abhilfe schaffen. Der Erfindung liegt die Aufgabe zugrunde, ein teilprothetisches Verbindungselement, insbesondere Geschiebe, für die Dentaltechnik, mit einer mindestens teilweisen Beschichtung der Metalloberfläche mit einer Haftvermittlerschicht zur Verbindung mit dentalen Kunststoffen zu schaffen, welches dank einer nahen chemischen Verwandtschaft zwischen Haftvermittlerschicht und dem für die Verbindung vorgesehen Dentalkunststoff eine extrem hohe Scherfestigkeit der Haftvermittlerschicht (von über 2000 N/cm$^2$) sowohl zum Metall als auch zum Dentalkunstoff garantiert und eine dauerhafte Bindung zwischen Dentalkunststoff und Metall ermöglicht, sowie imstande ist, hohe Kräfte, wie sie im dentalen Bereich auftreten, zu übertragen.

Erfindungsgemäß wird diese Aufgabe durch die Bereitstellung eines teilprothetischen Verbindungselementes aus Metall, welches die Merkmale des Anspruchs 1 aufweist und eines Verfahrens zur Herstellung des Verbindungselementes, welches die Merkmale des Anspruchs 3 aufweist, gelöst.

Das erfindungsgemäss beschichtete Verbindungselement weist die in der Praxis erforderlichen hohen Scherfestigkeitswerte auf. Die chemischen Einzelheiten der dabei verwendeten Haftvermittler sowie die ermittelten können der prioritätsgleichen Internationalen Anmeldung PCT/EP87/00656 mit der Internationalen Veröffeutlichungsnummer WO 88/03546 entnommen werden Haftwerte.

Die durch die Erfindung erreichten Vorteile sind im wesentlichen darin zu sehen, dass dank der erfindungsgemässen hohen Scherfestigkeit der Haftvermittlerschicht eine dauerhafte kraftübertragende Verbindung zwischen Metall und Dentalkunststoff erreicht wird. Die erfindungsgemässen Scherfestigkeitswerte liegen über 2000 N/cm$^2$ und können je nach Anwendung über 2400 N/cm$^2$ liegen, in Einzelfällen auch über 2700 N/cm$^2$.

Ein Ausführungsbeispiel der Erfindung, welches zugleich das Funktionsprinzip erläutert, ist im nachfolgenden Beispiel näher beschrieben.

Beispiel

Die abgestrahlten und entfetteten Aussenflächen der speziell geformten Gewindekappe (Patrize) eines Druckknopfankers werden in einem ersten herstellerseitigen Schritt in eine Polyäthylenfolie eingeschweisst In dieser Form wird die Gewindekappe vom Hersteller dem Zahntechniker geliefert.

Die Verarbeitung im zahntechnischen Labor verläuft dann wie folgt:

a) der fertig ausgearbeitete und polierte Modellguss wird an der Kontaktfläche zum Patrizenteil (Gewindekappe) mit Aluminiumoxid abgestrahlt;

b) die abgestrahlte Fläche wird mit Aceton enfettet;

c) die Gewindekappe wird aus ihrer Schutzfolie entnommen;

d) alle vorbehandelten Flächen (Modellguss und Gewindekappe) werden mit einem lichthärtenden Haftvermittler bestrichen, welcher auf einem Grundgemisch aus einem Methacryl-Gruppen aufweisenden flüssigen Monomer und einem Zusatz von Methacrylsäure basiert, das zusätzlich ein chlorosulfoniertes Polyolefin und ein fluoriertes Methacrylat enthält. Die Chemischen Einzelheiten dieses Haftvermittler können der obenerwähnten Internationalen Anmeldung entnommen werden;

e) die aufgebrachte Haftvermittlerschicht wird 8 Minuten mit UV-Licht bestrählt;

f) eine zweite Haftvermittlerschicht wird aufgetragen und ebenfalls 8 Minuten mit UV-Licht bestrahlt;

g) die Gewindekappe wird mit Zapit am Modellguss angeheftet (Positionsfixierung); und

h) die Modellgussprothese wird mit Prothesenkunststoff fertiggestellt.

## Patentansprüche

1. Teilprothetisches Verbindungselement aus Metall, insbesondere Geschiebe, für die Dentaltechnik, mit einer mindestens teilweisen Beschichtung der Metalloberfläche mit einer Haftvermittlerschicht zur Verbindung mit dentalen Kunststoffen, dadurch gekennzeichnet, dass die Haftvermittlerschicht aus einem ausgehärteten Grundgemisch aus Acryl-, Methacryl- oder Vinyl-Gruppen aufweisenden Monomeren, Monomerengemischen oder Präpolymeren und einem Zusatz von Acryl- oder Methacrylsäure besteht, das zusätzlich noch

A) ein halogensulfoniers Polyolefin und

B) ein halogeniertes Acrylat oder Methacrylat enthält.

2. Teilprothetisches Verbindungselement nach Anspruch 1, dadurch gekennzeichnet, dass die Haftvermittlerschicht auf einem Methacryl-Gruppen aufweisenden Monomer mit einem Zusatz von Methacrylsäure als Grundgemisch basiert, das zusätzlich ein chlorosulfoniertes Polyolefin und ein fluoriertes Methacrylat enthält.

3. Verfahren zur Herstellung des teilprothetischen Verbindungselementes nach Anspruch 1 oder 2, gekennzeichnet durch die folgenden Verfahrensschritte:

Schritt 1: Auftragung eines Grundgemisches, bestehend aus Acryl-, Methacryl- oder Vinyl-Gruppen aufweisenden flüssigen Monomeren, Monomerengemischen oder Präpolymeren und einem Zusatz von Acryl- oder Methacrylsäure, das zusätzlich noch

A) ein halogensulfoniertes Polyolefin und

B) ein halogeniertes Acrylat oder Methacrylat enthält,

auf die zu beschichtenden präparierten Metalloberflächen des Verbindungselementes;

Schritt 2: Aushärtung der aufgebrachten, flüssigen Haftvermittlerschicht.

4. Verfahren nach Anspruch 3, dadurch gekennzeichnet, dass die flüssige Haftvermittlerschicht mit Licht, vorzugsweise mit UV-Licht, ausgehärtet wird.

5. Verfahren nach Anspruch 3 oder 4, dadurch gekennzeichnet, dass nacheinander mehrere Haftvermittlerschichten aufgetragen und jeweils ausgehärtet werden.

6. Verfahren nach einem der Ansprüche 3 bis 5, dadurch gekennzeichnet, dass nach dem Verfahrenschritt 2 zusätzliche Metallteile, beispielsweise Gewindekappen, am Verbindungselement positionsmässig fixiert werden.

7. Verwenden des teilprothetischen Verbindungselementes nach Anspruch 1 oder 2 als kraftübertragendes dentalprothetisches Teil, insbesondere als Patrize, in einem Dentalgeschiebe.

## Revendications

1. Elémént de liaison de prothèse partielle en métal, plus particulièrement des attachments comportant au moins un revêtement partial de la surface de métal par une couche d'adjuvant d'adhérence en vue de la liaison à des matières plastiques dentaires, caractérisé en ce que la couche d'adjuvant d'adhérence se compose d'un mélange de base de prépolymères, de mélanges de monomères ou de monomères, présentant des radicaux acryle, méthacryle ou vinyle et d'une addition d'acide acrylique ou méthacrylique, qui contient complémentairement encore.

A) une polyoléfine halogénosulfonée et

B) un acrylate ou méthacrylate, halogéné.

2. Elément de liaison de prothèse partielle suivant la revendication 1, caractérisé en ce que la couche d'adjuvant d'adhérence est fondée sur un monomère présentant des radicaux méthacryl avec une addition d'acide méthacrylique, à titre de mélange de base, qui contient, complémentairement, une polyoléfine chlorosulfonée et un méthacrylate fluoré.

3. Procédé de fabrication d'un élément de liaison de prothèse partielle suivant la revendication 1 ou 2, caractérisé par les étapes opératoires suivantes:

Etape 1: Application d'un mélange de base, qui se compose de prépolymères, de mélanges de monomères, ou de monomères, liquides, présentant des radicaux acryle, méthacryle ou vinyle, qui contient complémentairement encore

A) une polyoléfine halogénosulfonée et

B) un acrylate ou méthacrylate, halogéné, sur les surfaces de métal préparées et à revêtir de l'élément de liaison.

Etape 2: Durcissement de la couche d'adjuvant d'adhérence, liquide, appliquée.

4. Procédé suivant la revendication 3 caractérisé en ce que l'on durcit la couche d'adjuvant d'adhérence, liquide, à l'aide de lumière, de préférence de lumière UV.

5. Procédé suivant la revendication 3 ou 4, caractérisé en ce que l'on applique de durcit chaque fois plusiers couches d'agent d'adhérence les unes après les autres.

6. Procédé suivant l'une des revendications 3 à 5, caractérisé en ce qu'après l'étape opératoire 2, on fixe selon la position des pièces métaliques supplémentaires, par exemple des coiffes filetées, à l'élément de liaison.

7. Utilisation de l'élément de liaison de prothèse partielle suivant la revendication 1 ou 2, à titre de pièce de prothèse transmettrice de force, plus particulièrement à titre de pièce mâle, dans un attachement dentaire.

## Claims

1. Partly-prosthetic metal connecting element, in particular dental attachment, for dental technology, of which at least one part of the metal surface is coated with a bonding agent layer for connection with dental plastics, characterized in that the bonding agent layer consists of a hardened basic mixture of monomers, monomeric mixtures or prepolymers containing acrylic, methacrylic or vinyl groups, with addition or acrylic or methacrylic acid, said basic mixture additionally containing

A) a halogen-sulfonated polyolefin, and

B) a halogenated acrylate or methacrylate.

2. Partly-prosthetic metal connecting element, characterized in that the bonding agent layer is based on a basic mixture of a monomer containing methacrylic groups, with addition of methacrylic acid, said basic mixture additionally containing a chloro-sulfonated polyolefin, and a fluorinated methacrylate.

3. Method for producing the partly-prosthetic metal connecting element according to claim 1 or 2, characterized by the following process steps:

Step 1: laying-on a basic mixture, consisting of monomers, monomeric mixtures or prepolymers containing acrylic, methacrylic or vinyl groups, with addition of acrylic or methacrylic acid, said basic mixture additionally containing

A) a halogen-sulfonated polyolefin, and

B) a halogenated acrylate or methacrylate on the prepared metal surfaces of the connecting element to be coated;

Step 2: hardening the laid-on, liquid bonding agent layer.

4. Method according to claim 3, characterized in that said liquid bonding agent layer is hardened by means of light, preferably by means of UV-light.

5. Method according to claim 3 or 4, characterized in that several bonding agent layers are successively laid-on and hardened.

6. Method according to one of the claims 3 to 5, characterized in that after execution of Step 2 additional metal parts are positionally fixed to the connecting element, for example screw caps.

7. Use of the partly-prosthetic connecting element according to claim 1 or 2 as load-transmitting dental-prosthetic element, in particular as a patrix, of a dental attachement.